# EUROPEAN PATENT APPLICATION

(11) **EP 3 566 707 A1**
(43) Date of publication of application: **13.11.2019**
(21) Application number: 17890044.5
(22) Date of filing: 04.08.2017
(51) Int. Cl.: A61K 31/7048, A61P 37/00, A61P 35/00, A61P 25/16, A61P 25/24, A61P 25/28, A61P 27/12

(54) **APPLICATION OF ALBIFLORIN AS INDOLEAMINE 2,3-DIOXYGENASE (IDO) INHIBITOR**

(30) Priority: 06.01.2017 CN 201710007295
(71) Applicant: Zhang, Zuoguang, Beijing 100013 (CN)
(72) Inventor: Zhang, Zuoguang, Beijing 100013 (CN)
(74) Representative: Harrison IP Limited
(86) International application number: PCT/CN2017/095987
(87) International publication number: WO 2018/126673

(57) **Abstract**

Use of albiflorin or a pharmaceutically acceptable salt thereof as an IDO inhibitor. Albiflorin or a pharmaceutically acceptable salt thereof as an IDO inhibitor can be used for the preparation of a drug, a food, a health care product, a food additive or a nutritional supplement for the prevention or treatment of diseases having a pathological feature involving tryptophan metabolic pathway mediated by overactivation of IDO, and these diseases are selected from one or more of cancer, depression, Alzheimer's disease, and Parkinson's disease.

## Description

### Technical Field

The invention pertains to the field of medicine, and relates to use of albiflorin, and more specifically, to use of albiflorin as an indoleamine 2,3-dioxygenase (IDO) inhibitor.

### Background Art

Indoleamine 2,3-dioxygenase (IDO) is a heme-containing enzyme in cells, and the only rate-limiting enzyme outside of liver that can catalyze the metabolism of tryptophan, leading to the decomposition of tryptophan through kynurenine pathway to generate a series of metabolites including quinolinic acid. Recent studies have fully demonstrated that overactivation of IDO leads to a tryptophan-deficient microenvironment *in vivo* through the degradation of tryptophan, and may further induce the occurrence of a variety of diseases closely related to tryptophan deficiency, such as cancer, depression, and Alzheimer's disease, etc.

Specifically, an increased IDO activity can inhibit T-cell proliferation by blocking T-cell activation through the degradation of tryptophan, thereby mediate tumor cells to avoid the attack of body's immune system, and increase body's incidence of cancer. Studies have shown that, 1-methyl tryptophan (1-MT), a currently well-established IDO inhibitor, can enhance the sensitivity of tumor cells to the immunostimulation of T-cells *in vitro,* retard the growth of tumor cells in *in vivo* animal model, and enhance the anti-tumor effects of chemotherapeutic drugs, and has inhibitory effect on nearly all spontaneous tumors (Friberg M, et al. Int J Cancer, 2002, 101: 151-155.). An IDO inhibitor was first used for the immunotherapy of tumor. In November 2015, Incyte Corporation, USA revealed that use of Epacadostat, a selective IDO inhibitor which was developed by this corporation in combination with Keytruda, an anti-PD-1 mAb of Merck Sharp Dohme Corporation showed good efficacy and safety in an early clinical trial.

Overactivation of IDO affects the normal metabolism of tryptophan, causing the abnormal increase of kynurenine in tryptophan metabolic pathway, and upregulation of level of quinolinic acid. Studies have demonstrated that the metabolic abnormality of this pathway is closely related to the inflammation of nervous system and occurrence of neurodegenerative diseases. Therefore, an IDO inhibitor can be used for the treatment of nervous system diseases, for example, Alzheimer's disease, Parkinson's disease, depression, cerebral infarction, etc. In the context of a total failure of the drug developments for the treatment of AD targeting Aβ-amyloid, the development of an IDO inhibitor and its use in the prevention and treatment of neurodegenerative diseases mediated by tryptophan-deficiency caused by overexpression of IDO, such as Alzheimer's disease, etc, has become a new global point of interest for the development of AD drugs.

Overactivation of IDO affects the normal metabolism of tryptophan and facilitates the inhibition of synthetic metabolic pathway of serotonin, and thereby may induce depression. Therefore, IDO is currently also a new target of significant attention for anti-depression in the pharmaceutical industry. The pathogenesis of depression has been unknown so far, and most of current anti-depression drugs were developed on the basis of Monoamine Hypothesis, of which the response rates after administration are only 50%∼60%, and all of them have adversary effects to some extent. According to the statistics, "less than 25% of the patients had received a continuous treatment of an antidepressant for 6 months, and a considerable proportion of them stopped the administration due to the adversary effects related to sex and sleep" (Andrew Solomon, Depression, P085). Due to lots of medical needs that have not been met, an attempt has continued to find anti-depression drugs with new biological targets and new mechanisms of action, wherein an IDO inhibitor is recently a major focus in the global medical industry.

Activation of IDO in clinically chronic inflammatory reaction is closely related to the development of depressive disorder, for example, the chronic Hepatitis C (HCV) patients treated by IFN-α have an incidence of depression of up to 25% to 33% during receiving the treatment; for the patients having brain stroke accompanied with inflammation, the cumulative incidence of post-stroke depressionr (PSD) in one year after stroke is 41.8%. There are two hypothesises regarding the mechanism of action of depressive disorder caused by activation of IDO which is facilitated by inflammation: one is tryptophan (TrP) depletion leading to the dysfunction of serotonergic (5-HT) neuron, and the other is the toxic effects increased by the neuron activated metabolites from kynurenine (KYN), causing over-activation of NMDA and generation of free radicals, and resulting in the pathologies of brain neurons in the particular central part. For depression and depressive disorder companied with somatopathy induced by overactivation of IDO resulted from inflammation, an IDO inhibitor may have a better therapeutic effect than SSRIs drugs such as Prozac, etc. For different causes of disease and different biological targets, a precise therapy of depression through targeted administration is one way to increase the therapeutic effect of depression.

### Contents of the Invention

The object of the invention is to provide a new use of albiflorin, and more specifically, relates to use of albiflorin as an indoleamine 2,3-dioxygenase (IDO) inhibitor. The invention also provides an indoleamine 2,3-dioxygenase (IDO) inhibitor, i.e., albiflorin, which can be used for the preparation of a drug, a food, a health care product, a food additive or a nutritional supplement for the prevention or treatment of diseases having a pathological feature involving tryptophan metabolic pathway mediated by IDO, and the diseases having a pathological feature involving tryptophan metabolic pathway mediated by IDO include the diseases such as cancer, depression, Alzheimer's disease, and Parkinson's disease, etc.

Albiflorin of the invention, having molecular formula of C₂₃H₂₈O₁₁, molecular weight of 480.46, and molecular structure as shown in Formula (I), is a monoterpenoid compound which is a natural active substance derived from the root of *Ranunculaceae,* such as *Paeonia lactiflora Pall* or *Paeonia veitchii Lynch,* or the root of *P. suffrsticosa Andrz.*

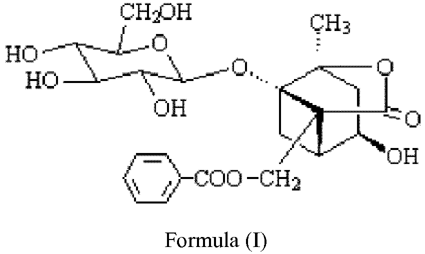

According to the use provided in the invention, the pharmaceutically acceptable salt of the compound shown by Formula (I) can be selected from one or more of citrate, hydrochloride, sulfate, malate, tartrate, citrate and phosphate.

The invention reveals that albiflorin or a pharmaceutically acceptable salt thereof is an IDO inhibitor. The IDO inhibitor of the invention, i.e., albiflorin or a pharmaceutically acceptable salt thereof can prevent or treat diseases having a pathological feature involving tryptophan metabolic pathway mediated by IDO through anti-inflammation or a direct inhibition of overexpression of IDO. This type of diseases include the cancers induced by the inhibition of immune system caused by overactivation of IDO, and the nervous system diseases mediated by the abnormality of tryptophan metabolic pathway induced by overactivation of IDO, such as depression, Alzheimer's disease, and Parkinson's disease, etc. The IDO inhibitor of the invention, i.e., albiflorin or a pharmaceutically acceptable salt thereof can also be used for the prevention and treatment of brain stroke accompanied with inflammation, post-stroke depression (PSD), and depressive disorders accompanied with somatopathy such as hepatitis, nephritis, and cancer, etc.

In one aspect, the invention provides use of albiflorin or a pharmaceutically acceptable salt thereof, or an extract containing albiflorin or a pharmaceutically acceptable salt thereof as an IDO inhibitor.

The invention also provides use of albiflorin or a pharmaceutically acceptable salt thereof, or an extract containing albiflorin or a pharmaceutically acceptable salt thereof in the preparation of a drug, a food, a health care product, a food additive or a nutritional supplement for inhibiting the activity of IDO.

The invention also provides use of albiflorin or a pharmaceutically acceptable salt thereof, or an extract containing albiflorin or a pharmaceutically acceptable salt thereof in the preparation of a drug, a food, a health care product, a food additive or a nutritional supplement for the prevention or treatment of a disease having a pathological feature involving tryptophan metabolic pathway mediated by overactivation of IDO.

Preferably, the disease is selected from one or more of AIDS, cancer, neurodegenerative disease, depression, cataract, age-related yellowing, and autoimmune disease.

More preferably, the neurodegenerative disease is selected from one or more of Alzheimer's disease, Huntington's disease and Parkinson's disease.

Further preferably, the drug is a drug used for the immunotherapy of cancer.

The invention also provides use of albiflorin or a pharmaceutically acceptable salt thereof, or an extract containing albiflorin or a pharmaceutically acceptable salt thereof in the preparation of a drug, a food, a health care product, a food additive or a nutritional supplement for the prevention or treatment of inflammatory depression, post-stroke depression (PSD), depressive disorder accompanied with somatopathy such as cancer, hepatitis, nephritis, etc., stroke accompanied with inflammation, Alzheimer's disease accompanied with inflammation, or Parkinson's disease accompanied with inflammation.

In another aspect, the invention provides a method for inhibiting overactivation of IDO, the method comprising administration of a therapeutically effective amount of albiflorin or a pharmaceutically acceptable salt thereof, or an extract containing albiflorin or a pharmaceutically acceptable salt thereof.

The invention also provides a method for the prevention or treatment of a disease having a pathological feature involving tryptophan metabolic pathway mediated by overactivation of IDO, the method comprising administration of a therapeutically effective amount of albiflorin or a pharmaceutically acceptable salt thereof, or an extract containing albiflorin or a pharmaceutically acceptable salt thereof.

Preferably, the disease is selected from one or more of AIDS, cancer, neurodegenerative disease, depression, cataract, age-related yellowing, and autoimmune disease.

More preferably, the neurodegenerative disease is selected from one or more of Alzheimer's disease, Huntington's disease and Parkinson's disease.

Further preferably, the method is an immunotherapy of cancer.

The invention also provides a method for the prevention or treatment of inflammatory depression, post-stroke depression (PSD), depressive disorder accompanied with somatopathy such as cancer, hepatitis, nephritis, etc., stroke accompanied with inflammation, Alzheimer's disease accompanied with inflammation, or Parkinson's disease accompanied with inflammation, the method comprising administration of a therapeutically effective amount of albiflorin or a pharmaceutically acceptable salt thereof, or an extract containing albiflorin or a pharmaceutically acceptable salt thereof.

In still another aspect, the invention also provides a composition for inhibiting IDO, comprising albiflorin or a pharmaceutically acceptable salt thereof, or an extract containing albiflorin or a pharmaceutically acceptable salt thereof.

Preferably, the composition is a drug, a food, a health care product, a food additive or a nutritional supplement.

The invention also provides a composition for the prevention or treatment of a disease having a pathological feature involving tryptophan metabolic pathway mediated by overactivation of IDO, comprising albiflorin or a pharmaceutically acceptable salt thereof, or an extract containing albiflorin or a pharmaceutically acceptable salt thereof.

Preferably, the composition is a drug, a food, a health care product, a food additive or a nutritional supplement.

Preferably, the disease is selected from one or more of AIDS, cancer, neurodegenerative disease, depression, cataract, age-related yellowing, and autoimmune disease.

More preferably, the neurodegenerative disease is selected from one or more of Alzheimer's disease, Huntington's disease and Parkinson's disease.

Further preferably, the composition is used for the immunotherapy of cancer.

The invention also provides a composition for the prevention or treatment of inflammatory depression, post-stroke depression (PSD), depressive disorder accompanied with somatopathy such as cancer, hepatitis, nephritis, etc., stroke accompanied with inflammation, Alzheimer's disease accompanied with inflammation, or Parkinson's disease accompanied with inflammation, comprising albiflorin or a pharmaceutically acceptable salt thereof, or an extract containing albiflorin or a pharmaceutically acceptable salt thereof.

In still another aspect, the invention also provides albiflorin or a pharmaceutically acceptable salt thereof, or an extract containing albiflorin or a pharmaceutically acceptable salt thereof used as an IDO inhibitor.

The invention also provides albiflorin or a pharmaceutically acceptable salt thereof, or an extract containing albiflorin or a pharmaceutically acceptable salt thereof used for the prevention or treatment of a disease having a pathological feature involving tryptophan metabolic pathway mediated by overactivation of IDO.

Preferably, the disease is selected from one or more of AIDS, cancer, neurodegenerative disease, depression, cataract, age-related yellowing, and autoimmune disease.

More preferably, the neurodegenerative disease is selected from one or more of Alzheimer's disease, Huntington's disease and Parkinson's disease.

Further preferably, albiflorin or a pharmaceutically acceptable salt thereof, or an extract containing albiflorin or a pharmaceutically acceptable salt thereof is used for the immunotherapy of cancer.

The invention also provides albiflorin or a pharmaceutically acceptable salt thereof, or an extract containing albiflorin or a pharmaceutically acceptable salt thereof for the prevention or treatment of inflammatory depression, post-stroke depression (PSD), depressive disorder accompanied with somatopathy such as cancer, hepatitis, nephritis, etc., stroke accompanied with inflammation, Alzheimer's disease accompanied with inflammation, or Parkinson's disease accompanied with inflammation.

The invention has found, in the study of chronic stress rat model (CUMS) and mouse inflammatory depression-like behavior induced by lipopolysaccharide (LPS), that the inflammation induced by both chronic stress and LPS can cause overexpression of IDO, while albiflorin or a pharmaceutically acceptable salt thereof of the invention, as an IDO inhibitor, can decrease the ratio of kynurenine/tryptophan through anti-inflammation and a direct inhibition of overexpression of IDO, and thus act as an IDO inhibitor which is used for the treatment of a disease having a pathological feature involving tryptophan metabolic pathway mediated by overactivation of IDO.

Furthermore, the invention has found that even though albiflorin and fluoxetine, a positive drug, are both anti-depressive, their mechanisms of anti-depression are different: albiflorin is an IDO inhibitor which is anti-depressive by inhibiting overexpression of IDO through decreasing the ratio of kynurenine/tryptophan (KYN/TrP) in plasma and hippocampus region; and the positive drug fluoxetine is not an IDO inhibitor, which is anti-depressive not by inhibiting IDO and regulating tryptophan metabolic pathway. Sustained immune response is an important reason resulting in refractory and recurrent depression. However, albiflorin of the invention as an IDO inhibitor has a better therapeutic effect than SSRIs drugs such as fluoxetine, etc. in the treatment of inflammatory depression, depression of stroke patient accompanied with inflammation, post-stroke depression (PSD), depressive disorder accompanied with somatopathy such as hepatitis and nephritis, etc., and depressive disorder accompanied with cancer mediated by overactivation of IDO.

Compared with the prior art, the IDO inhibitor of the invention albiflorin can be used in a precise treatment with a better safety and efficacy for diseases having a pathological feature involving tryptophan metabolic pathway mediated by overactivation of IDO:
1. The IDO inhibitor of the invention albiflorin enhances the immunologic function of T-cells and prevents tumor cells from avoiding the monitoring and killing of body's immune system by inhibiting overactivation of IDO *in vivo,* and can be used in the preparation of an anti-cancer drug for immunotherapy.
2. The IDO inhibitor of the invention albiflorin prevents and slows the occurrence and development of Alzheimer's disease by inhibiting overactivation of IDO *in vivo* and reducing the generation of kynurenine and quinolinic acid from tryptophan, and can be made into a drug having a new mechanism of action for anti-aging and treating neurodegenerative diseases such as Alzheimer's disease, etc.
3. The IDO inhibitor of the invention albiflorin is more suitable for the preparation of a new-generation of anti-depression drug for a precise therapy of depression, post-stroke depression (PSD), depressive disorder accompanied with somatopathy such as cancer, hepatitis, nephritis, and cancer, etc. induced by overactivation of IDO.
4. The IDO inhibitor of the invention albiflorin is a pure natural drug, and although its binding mode is similar with that of an IDO chemical inhibitor, there's a difference that an artificially synthesized chemical inhibitor binds to the iron ion of IDO through the nitrogen atom, while albiflorin binds to the iron ion of IDO through the oxygen atom. This structural difference determines that the natural IDO inhibitor of the invention albiflorin has a higher safety.

### Brief Description of the Drawings

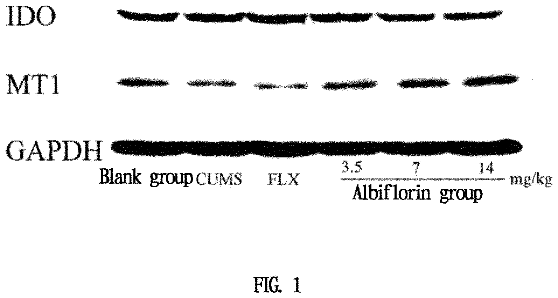

FIG. 1 shows the effects of albiflorin of the invention on IDO of the hippocampal tissue and the melatonin receptors (MT1) in the chronic stressed rats.

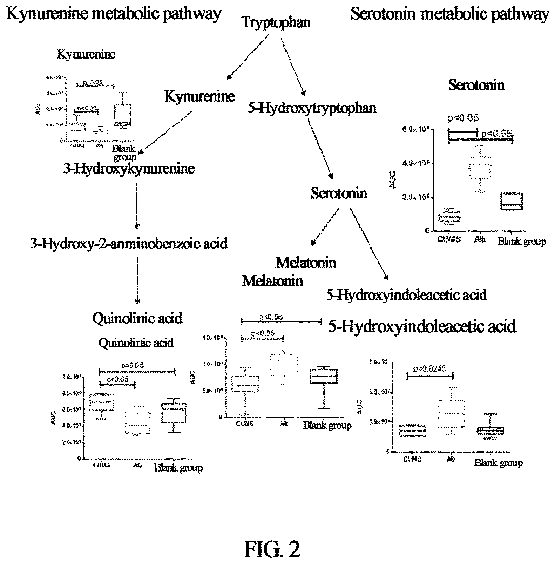

FIG. 2 is a scheme of metabolic pathway in which kynurenine-quinolinic acid is downregulated and serotonin-melatonin is upregulated through inhibiting IDO by albiflorin of the invention.

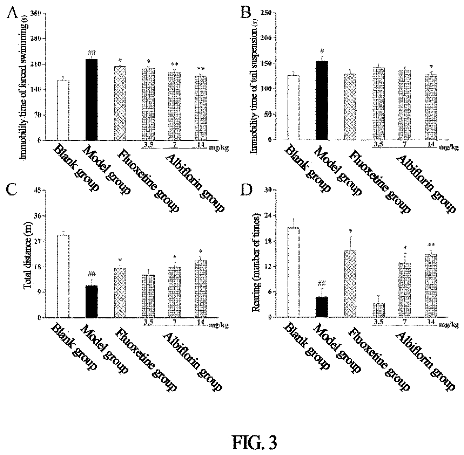

FIG. 3 shows the effects of albiflorin of the invention in the mouse praxiology tests after modeling with LPS: Figure A shows the immobility time of the mice in the Forced Swimming Test; Figure B shows the immobility time of the mice in the Tail Suspension Test; Figure C shows the total movement distance of the mice in the Open Field Test; and Figure. D shows the number of rearings of the mice in the Open Field Test.

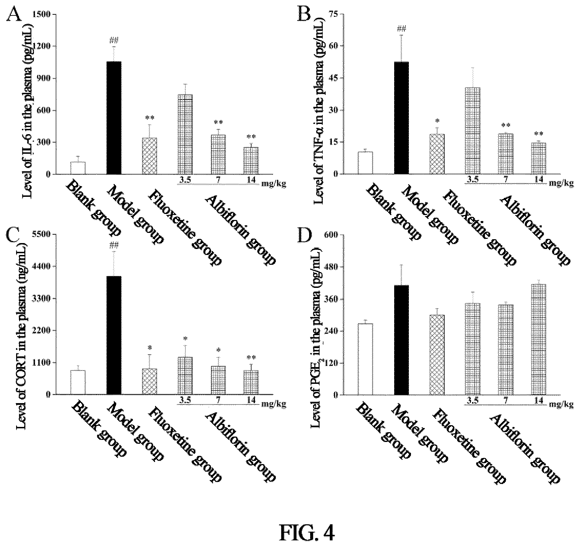

FIG. 4 shows the effects of albiflorin of the invention on the levels of inflammatory factors in the mouse plasma after modeling with LPS: Figure A shows the level of IL-6 in the mouse plasma; Figure B shows the level of TNF-α in the mouse plasma; Figure C shows the level of CORT in the mouse plasma; and Figure D shows the level of PGE₂ in the mouse plasma.

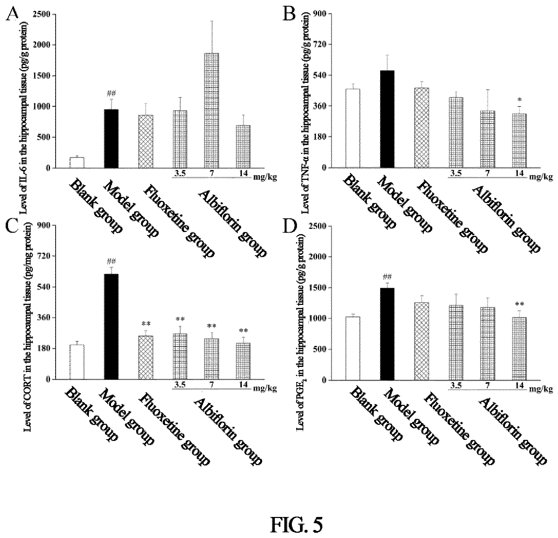

FIG. 5 shows the effects of albiflorin of the invention on the levels of the inflammatory factors in the mouse hippocampal tissue after modeling with LPS: Figure A shows the level of IL-6 in the mouse hippocampal tissue; Figure B shows the level of TNF-α in the mouse hippocampal tissue; Figure C shows the level of CORT in the mouse hippocampal tissue; and Figure D shows the level of PGE₂ in the mouse hippocampal tissue.

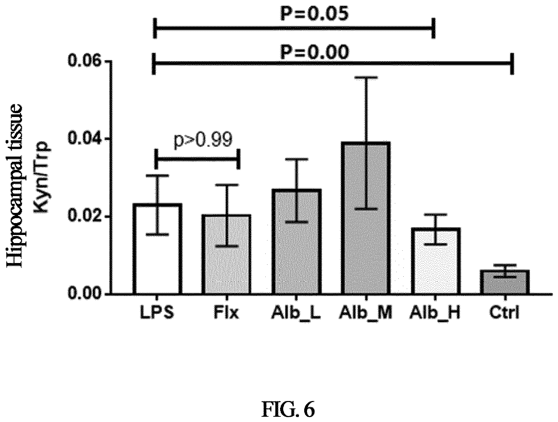

FIG. 6 shows the effects of albiflorin of the invention on the content ratio of kynurenine (KYN) to tryptophan (TrP) and the IDO activity in the mouse hippocampal tissue after modeling with LPS.

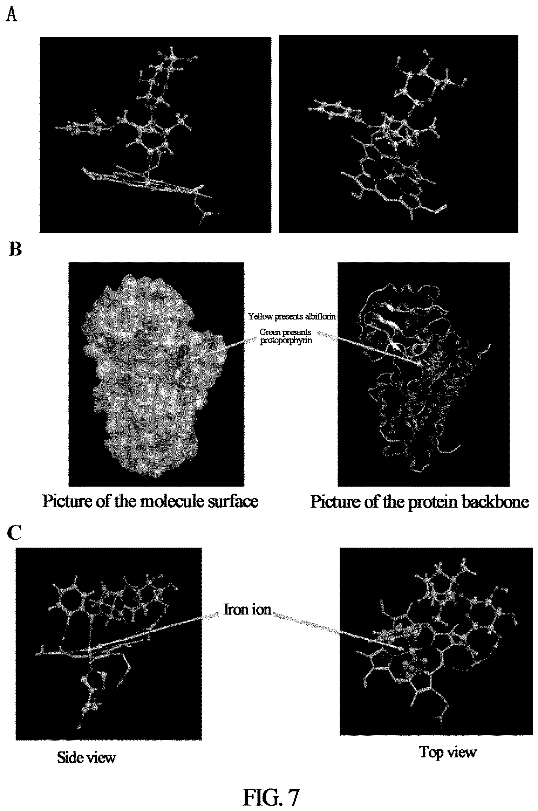

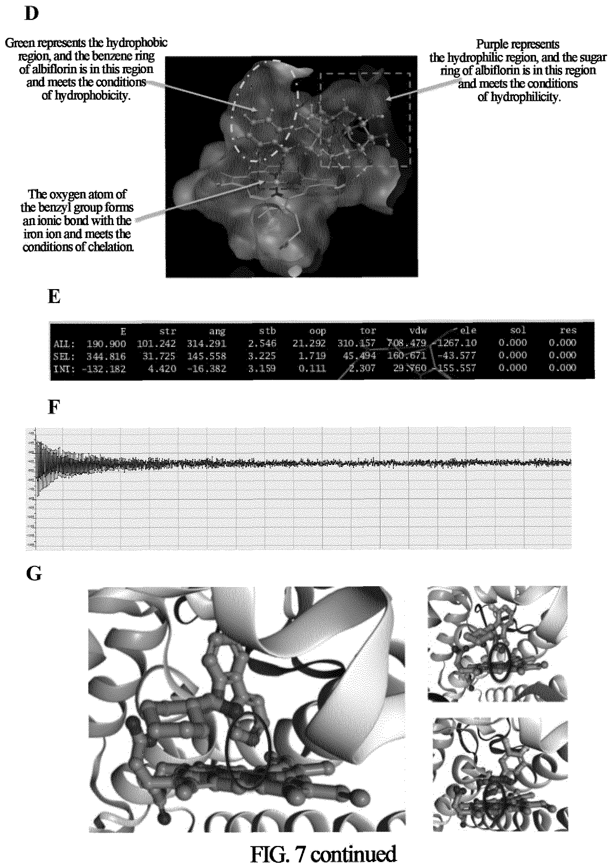

FIG. 7 shows the experimental results of docking albiflorin of the invention to IDO1 protein molecule using MOE2016 software: Figure A shows the docking result of the first phase; Figure B shows the site in IDO1 where albiflorin binds; Figure C shows the binding mode of albiflorin to heme in IDO1; Figure D shows the binding mode of albiflorin to IDO1 activity pocket; Figure E shows the calculation result of the binding free energy of albiflorin and IDO1; Figure F is a graph of dynamic simulation of potential energy; and Figure G shows the binding mode of IDO1 to some artificial synthetic inhibitors.

### Best Mode for Carrying Out the Invention

The invention will be further illustrated in connection with the specific examples. The following examples are presented only for the illustration of the specific uses of the present invention in treating depression and improving cognitive function, not for the limitation of the application scope of the present invention.

### Example 1: Effects of the IDO inhibitor of the invention albiflorin on overexpression of IDO and depression-like behaviors induced in the chronic stressed rat model (CUMS)

1. In the hippocampal tissue of the rat as chronic unpredictable mild stress model (CUMS), the secretion of IDO increased significantly, and the secretion of melatonin receptors 1 (MT1) decreased significantly. After 7 days of administration of albiflorin (7 mg/day, 14 mg/day), the IDO activity in the rat hippocampus was significantly inhibited (P<0.01), and the melatonin receptors 1 (MT1) in the rat hippocampus were increased significantly (P<0.01). The results are found in the table below. Western Blot was used to detect the effects of albiflorin on IDO and MT1, and the results are found in FIG. 1:

**Effects of albiflorin on indoleamine 2,3-dioxygenase (IDO) and melatonin receptors 1 (MT1) in the rat hippocampal tissue (Mean ± SE)**

| Group | IDO | MT1 |
|---|---|---|
| | (folds relative to Blank group) | (folds relative to Blank group) |
| Blank group | 1.00 ± 0.010** | 1.0±0.010^{##} |
| Model group | 1.21 ± 0.011 | 0.68±0.007 |
| Fluoxetine (10 mg/kg) | 1.29 ± 0.014^{#} | 0.50±0.005** |
| Albiflorin (3.5 mg/kg) | 1.46 ± 0.014^{##} | 1.37±0.013^{##} |
| Albiflorin (7 mg/kg) | 1.02 ± 0.010 ** | 1.39±0.0014^{##} |
| Albiflorin (14 mg/kg) | 0.80 ± 0.008** | 1.39±0.014^{##} |

| | | |
|---|---|---|
| (# represents an increase relative to Model group P<0.05, ## represents an increase relative to Model group P<0.01, ** represents a decrease relative to Model group P<0.01) | | |

2. The synthetic metabolic pathway of serotonin in the hippocampal tissue of the rat as chronic unpredictable mild stress model (CUMS) was inhibited, while the metabolic pathway of kynurenine and quinolinic acid was upregulated (P<0.01). After 7 days of administration of albiflorin (7 mg/kg, 14 mg/kg), the levels of kynurenine and quinolinic acid in the rat hippocampus was decreased significantly (P < 0.01), indicating that kynurenine metabolic pathway was downregulated; the levels of serotonin and melatonin in the hippocampus were increased significantly (P<0.01), indicating that serotonin and melatonin metabolic pathway was upregulated; and the ratio of kynurenine to tryptophan (KYN/TrP) was downregulated significantly, indicating that the IDO activity was inhibited (see FIG. 2).

### Example 2: Effects of the IDO inhibitor of the invention albiflorin on LPS-induced IDO activation, mouse inflammatory depression-like behaviors and cognitive dysfunction

The mice were randomly divided into 6 groups (16 for each group): Blank group, Model group, Fluoxetine hydrochloride (a positive drug) group (10 mg/kg), Low dose of albiflorin group (3.5 mg/kg), Medium dose of albiflorin group (7 mg/kg), and High dose of albiflorin group (14 mg/kg), and administered continuously by gavage for 7 days.

### I. Tests and detection contents

1. The behavioral tests: Forced Swimming Test (FST), Tail Suspension Test (TST) and Open Field Test (OFT)
   On the 7^{th} day after administration, LPS was injected intraperitoneally (0.83 mg/kg) for modeling. At 16 h after the injection, the Open Field Test (OFT) was carried out; at 24 h after the injection, the Forced Swimming Test (FST) and Tail Suspension Test (TST) were carried out. Thereafter, the blood was taken by enucleating eyeball, and centrifuged to collect the plasma. After the mice were sacrificed, the hippocampal tissues were taken out and quickly frozen in liquid nitrogen, and stored at -80°C.
2. Determination of the effects of albiflorin and the positive drug fluoxetine on the concentrations of inflammatory factors IL-6, TNF-α, and PGE2, and glucocorticoid CORT in the mouse plasma and hippocampal tissue.
3. Determination of the effects of albiflorin and fluoxetine on expression of IDO in the mouse hippocampal tissue.

### II. Statistical analysis

The test data are shown in mean ± SE, and originPro8.0 software was used for the statistical analysis of the data and plotting. One-Way ANOVA method was used for the comparison between the groups, and P<0.05 and P<0.01 were regarded as having a significant difference.

### III. Test results

### 1. Mouse behavioral tests after modeling with LPS

The behavioral test results: after modeling by intraperitoneal injection of LPS into the mice, a depression-like status occurred. In each of the administration groups, the mouse depression-like status induced by LPS can be alleviated to different extents, and the immobility times in the Forced Swimming Test and Tail Suspension Test were reduced, wherein there's a significant difference between the immobility times of the mice in High dose of albiflorin group in the Forced Swimming Test and the Tail Suspension Test compared with those in Model group (see FIG. 3).

### 2. Levels of inflammatory factors in the mouse plasma

The experimental detection results showed that: the levels of inflammatory factors IL-6 and TNF-α, and glucocorticoid CORT in the plasma of the mouse in Model group were substantially increased compared with those in Blank control group, demonstrating that an inflammatory reaction and hyperfunction of HPA axis were generated in the mice after the intraperitoneal injection of LPS. In the administration groups of medium and high doses of albiflorin, the levels of inflammatory factors IL-6 and TNF-α in the mouse plasma can be substantially decreased, and the inflammatory reaction induced by LPS was alleviated, demonstrating that albiflorin has a good anti-inflammatory effect. Meanwhile, albiflorin can substantially decrease the concentration of CORT in the mouse plasma, and has the function of reverse regulation of hyperfunctional HPA axis (see FIG. 4).

### 3. Levels of inflammatory factors in the mouse hippocampal tissue

An ELISA assay was used to detect the levels of inflammatory factors IL-6, TNF-α and PGE2, and CORT in the mouse hippocampal tissue. The results showed that the levels of inflammatory factors and CORT in the mouse hippocampal tissue in Model group were substantially increased compared with those in Blank control group, demonstrating that the concentrations of inflammatory factors in the mouse brain tissue were increased after the intraperitoneal injection of LPS, meanwhile HPA axis was hyperfunctional, causing dysfunction. The levels of inflammatory factors IL-6 and TNF-α in the mouse hippocampal tissue in High dose of albiflorin group were significantly decreased compared with those in Model group, indicating that albiflorin has an anti-inflammatory effect. The level of CORT in the hippocampal tissue was substantially decreased, indicating that albiflorin can reversely regulate the hyperfunctional HPA axis (see FIG. 5).

### 4. Ratio of kynurenine (KYN)/tryptophan (TrP) in the mouse hippocampal tissue

The activity of IDO in the hippocampus was expressed through the levels of KYN and TrP and the ratio of KYN/TrP. LPS induced an inflammatory reaction in the mice, leading to a substantial increase of the ratio of kynurenine/tryptophan (KYN/TrP) in the mouse hippocampal tissue in Model group compared with that in Blank control group, indicating that IDO in the hippocampus was overexpressed. Compared with Model group, the activation of IDO in the hippocampus induced by LPS failed to be corrected in the positive drug Fluoxetine administration group, while the ratio of kynurenine/tryptophan (KYN/TrP) in High dose of albiflorin group was decreased (P<0.05), and the activity of IDO was significantly inhibited (see FIG. 6).

Through detection of the KYN/TrP ratio in the LPS mouse hippocampal tissue, this study found that a high dose of albiflorin (14 mg/kg) could decrease the IDO activity in the mouse hippocampal tissue caused by the LPS-induced inflammatory reaction, while the LPS-induced overexpression of IDO failed to be corrected in the positive drug Fluoxetine administration group (10 mg/kg).

The test results demonstrated that, even though albiflorin and a positive drug fluoxetine are both anti-depressive, their anti-depressive mechanisms are different: albiflorin as an IDO inhibitor is anti-depressive by inhibiting overexpression of IDO through decreasing the ratios of kynurenine/tryptophan (KYN/TrP) in the plasma and the hippocampus region; the positive drug fluoxetine is not an IDO inhibitor, which is anti-depressive not by inhibiting IDO and regulating tryptophan metabolic pathway. Sustained immune response is an important reason resulting in refractory and recurrent depression. Albiflorin as an IDO inhibitor may have a better therapeutic effect than SSRI-type drugs in the treatment of depression and depressive disorder accompanied with the inflammatory somatopathy mediated by IDO, and can be used for a precise therapy of depression by mediated by IDO.

### Analysis of the two examples (Example 1 and Example 2)

The invention found and demonstrated that albiflorin is an IDO inhibitor through behavioral studies in Example 1 (CUMS experiment) and Example 2 (LPS experiment), targeted metabonomics study and biological target detection.
1. The IDO inhibitor of the invention albiflorin reduces the generation of kynurenine and quinolinic acid from tryptophan, and promotes the generation of 5-HT and melatonin from tryptophan through the serotonin pathway through inhibiting upregulation of IDO in blood and hippocampus induced by stress and inflammation, can be made into an anti-depression drug, and used for the prevention or treatment of depression, particularly for the treatment of inflammatory depression and depressive disorder accompanied with various infectious or non-infectious inflammatory somatopathies.
2. The IDO inhibitor of the invention albiflorin enhances the immunologic function of T-cells and prevents tumor cells from avoiding the monitoring and killing of body's immune system by inhibiting overactivation of IDO *in vivo,* and can be prepared into an anti-cancer drug for the immunotherapy of tumors.
3. The IDO inhibitor of the invention albiflorin prevents and slows the occurrence and development of Alzheimer's disease by inhibiting overactivation of IDO *in vivo* and reducing the generation of kynurenine and quinolinic acid from tryptophan, and can be made into a drug for anti-aging and treating neurodegenerative diseases such as Alzheimer's disease, etc.
4. According to the invention, albiflorin and a pharmaceutically acceptable salt thereof, or an albiflorin-containing extract or composition can be developed into a drug, a food, a health care product, a food additive or a nutritional supplement for the treatment, prevention, regulation, and improvement of diseases or sub-health statuses having a pathological feature of dysfunction in tryptophan metabolic pathway mediated by IDO.

### Example 3: Experiment of docking the IDO1 inhibitor of the invention albiflorin to IDO1 protein molecule using MOE2016 software

### 1. Selection of the crystal structure of IDO1

So far 20 IDO protein crystal structures have been reported. After consideration of multiple factors such as biological sources, resolution, ligand small molecule structure, etc., eventually a crystal structure with serial number 4PK5 was determined as the molecular structure docking to IDO1 protein.

### 2. The small molecule structure of albiflorin is as follow:

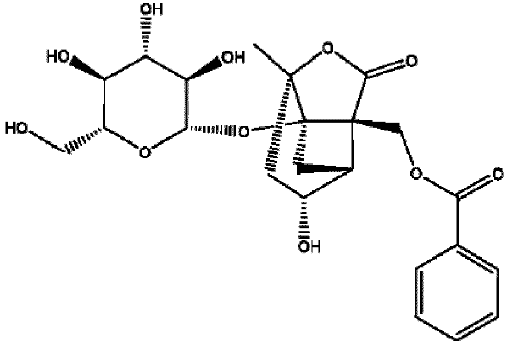

### 3. Molecular docking experiment

MOE2016 software package was used to complete the docking experiment and other related calculations.

### Procedure:

A receptor structure was prepared, the errors in the structure were corrected, and the partial charge calculation and protonation process were completed. A suitable subunit was selected as the receptor, and the rest was deleted.

Using Flexible Alignment function of the MOE software, and taking the original ligand for the protein as the template, the fitting mode between albiflorin and the ligand was investigated, and more than 20 fitting results were obtained.

The fitting results were examined one by one, of which the relatively reasonable fitting results were selected and optimized to generate the docking result of the first phase (see FIG. 7A).

Based on the docking result of the first phase, Dock module of the MOE software was used to perform the secondary automatic docking to obtain the second batch of docking results, from which the reasonable results were further screened.

The results from the two phases were subject to a structural optimization, and a molecular dynamic simulation method was used to test the stability of the docking results, the simulation time being 6 ns.

Eventually the binding mode of albiflorin to IDO1 was determined, and the binding free energy was calculated.

### Result and Discussion

Albiflorin binds to the core region of IDO1, close to the heme structure (composed of iron ion and protoporphyrin). The activity pocket of receptor is composed of several alpha helical structures, inside of which heme is, and there's a relatively bigger entry towards the environment outside of the protein (see FIG. 7B).

Albiflorin together with protoporphyrin and histidine His346 forms a chelating system, which wraps iron ion in the middle. Iron ion is the key factor for the catalytic reaction of IDO1. Albiflorin binding to iron ion occupied the activity pocket successfully, and thereby resulted in a competitive inhibition (see FIG. 7C).

Besides binding to iron ion, the other two parts of albiflorin also match with the polarity of the receptor pocket. A comprehensive consideration of these three points, albiflorin meets the requirements of an IDO1 inhibitor. The binding mode obtained by us is also reasonable (see FIG. 7D).

We used MOE to perform a preliminary free energy calculation of the docking results of the second phase, and the result showed that the binding free energy was -132 kcal/mol. This result demonstrated in terms of order of magnitude that there is a higher binding free energy between albiflorin and IDOl, and thereby speculate that there is a higher inhibitory activity (see FIG. 7E).

We used MOE to perform a dynamic simulation of the residues within a range of 6 angstroms from the activity pocket indicated by the docking results. The results showed that the docking results were stable and didn't vary over time (see FIG. 7F).

All of several chemical inhibitors of IDO1 that have currently been reported bind in the same mode (see FIG. 7G). The difference is that all of them bind to iron ion through nitrogen atom, while albiflorin bind to iron ion through oxygen atom. The binding free energy of oxygen atom is lower that of nitrogen atom. Therefore, as compared with these chemical inhibitors, albiflorin may have a lower binding intensity and a weaker inhibitory activity.

The above description of the specific embodiments of the invention does not limit the invention, and various modifications and variations may be made by those of skills in the art according to the invention without departing from the spirit of the invention, and are all within the scope of the appended claims of the invention.

## Claims

1. Use of albiflorin or a pharmaceutically acceptable salt thereof, or an extract containing albiflorin or a pharmaceutically acceptable salt thereof as an IDO inhibitor.

2. Use of albiflorin or a pharmaceutically acceptable salt thereof, or an extract containing albiflorin or a pharmaceutically acceptable salt thereof in the preparation of a drug, a food, a health care product, a food additive or a nutritional supplement for inhibiting the activity of IDO.

3. Use of albiflorin or a pharmaceutically acceptable salt thereof, or an extract containing albiflorin or a pharmaceutically acceptable salt thereof in the preparation of a drug, a food, a health care product, a food additive or a nutritional supplement for the prevention or treatment of a disease having a pathological feature involving tryptophan metabolic pathway mediated by overactivation of IDO;
preferably, the disease is selected from one or more of AIDS, cancer, neurodegenerative disease, depression, cataract, age-related yellowing, and autoimmune disease;
more preferably, the neurodegenerative disease is selected from one or more of Alzheimer's disease, Huntington's disease and Parkinson's disease;
further preferably, the drug is a drug used for the immunotherapy of cancer.

4. Use of albiflorin or a pharmaceutically acceptable salt thereof, or an extract containing albiflorin or a pharmaceutically acceptable salt thereof in the preparation of a drug, a food, a health care product, a food additive or a nutritional supplement for the prevention or treatment of inflammatory depression, post-stroke depression (PSD), depressive disorder accompanied with somatopathy such as cancer, hepatitis, nephritis, etc., stroke accompanied with inflammation, Alzheimer's disease accompanied with inflammation, or Parkinson's disease accompanied with inflammation.

5. A method for inhibiting overactivation of IDO, comprising administration of a therapeutically effective amount of albiflorin or a pharmaceutically acceptable salt thereof, or an extract containing albiflorin or a pharmaceutically acceptable salt thereof.

6. A method for the prevention or treatment of a disease having a pathological feature involving tryptophan metabolic pathway mediated by overactivation of IDO, comprising administration of a therapeutically effective amount of albiflorin or a pharmaceutically acceptable salt thereof, or an extract containing albiflorin or a pharmaceutically acceptable salt thereof;
preferably, the disease is selected from AIDS, cancer, neurodegenerative disease, depression, cataract, age-related yellowing, and autoimmune disease;
more preferably, the neurodegenerative disease is selected from one or more of Alzheimer's disease, Huntington's disease and Parkinson's disease;
further preferably, the method is the immunotherapy of cancer.

7. A method for the prevention or treatment of inflammatory depression, post-stroke depression (PSD), depressive disorder accompanied with somatopathy such as cancer, hepatitis, nephritis, etc., stroke accompanied with inflammation, Alzheimer's disease accompanied with inflammation, or Parkinson's disease accompanied with inflammation, comprising administration of a therapeutically effective amount of albiflorin or a pharmaceutically acceptable salt thereof, or an extract containing albiflorin or a pharmaceutically acceptable salt thereof.

8. A composition for inhibiting IDO, comprising albiflorin or a pharmaceutically acceptable salt thereof, or an extract containing albiflorin or a pharmaceutically acceptable salt thereof;
preferably, the composition is a drug, a food, a health care product, a food additive or a nutritional supplement.

9. A composition for the prevention or treatment of a disease having a pathological feature involving tryptophan metabolic pathway mediated by overactivation of IDO, comprising albiflorin or a pharmaceutically acceptable salt thereof, or an extract containing albiflorin or a pharmaceutically acceptable salt thereof;
preferably, the composition is a drug, a food, a health care product, a food additive or a nutritional supplement;
preferably, the disease is selected from one or more of AIDS, cancer, neurodegenerative disease, depression, cataract, age-related yellowing, and autoimmune disease;
more preferably, the neurodegenerative disease is selected from one or more of Alzheimer's disease, Huntington's disease and Parkinson's disease;
further preferably, the composition is used for the immunotherapy of cancer.

10. A composition for the prevention or treatment of inflammatory depression, post-stroke depression (PSD), depressive disorder accompanied with somatopathy such as cancer, hepatitis, nephritis, etc., stroke accompanied with inflammation, Alzheimer's disease accompanied with inflammation, or Parkinson's disease accompanied with inflammation, comprising albiflorin or a pharmaceutically acceptable salt thereof, or an extract containing albiflorin or a pharmaceutically acceptable salt thereof.
